# EUROPÄISCHE PATENTSCHRIFT

(11) **EP 2 181 724 B1**
(45) Veröffentlichungstag und Bekanntmachung des Hinweises auf die Patenterteilung: **08.05.2013**
(21) Anmeldenummer: 09012744.0
(22) Anmeldetag: 08.10.2009
(51) Int. Cl.: A61M 1/16

(54) **Fluidsystem**
Fluid system
Système fluidique

(30) Priorität: 04.11.2008 DE 102008055754
(43) Veröffentlichungstag der Anmeldung: 05.05.2010
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- DE-A1- 2 856 664
- DE-A1- 10 256 584
- DE-B3-102006 028 764
- DE-U1- 20 013 555
- FR-A- 1 586 103
- US-A- 5 585 003

## Beschreibung

Die Erfindung betrifft ein Fluidsystem mit einer Hauptversorgungsleitung und wenigstens einer davon abzweigenden, zu Verbrauchern führenden Sekundärleitung. Allgemein ist die Erfindung bei Systemen verwendbar, bei denen eine Hauptversorgungsleitung aus räumlichen bzw. konstruktiven Gründen oder aus Kostengründen nicht direkt zum Verbraucher geführt werden kann, weshalb eine oder mehrere Sekundärleitungen von der Hauptversorgungsleitung abzweigen. Bei dem Fluid, das in dem Leitungssystem geführt wird, kann es sich um ein gasförmiges Medium handeln, beispielsweise um sterile Luft, oder um eine Flüssigkeit. Die Erfindung ist insbesondere für Dialysestationen geeignet, bei der die Verbraucher, d.h. die Dialysegeräte, mit kontaminationsfreiem Wasser versorgt werden müssen. Die Erfindung ist aber ebenso beispielsweise für die Wasserversorgung in der Pharmaindustrie oder für eine Laborwasserversorgung verwendbar.

Von der Hauptversorgungsleitung kann jeweils eine einzige Sekundärleitung zu einem Verbraucher führen, die damit eine Stichleitung ist, die in Stillstandphasen bzw. Ruhepausen der Versorgung nicht durchströmt wird. Es können aber auch zwei Sekundärleitungen zu einem Verbraucher führen, die eine Sekundärringleitung bilden, bei der durch eine Sekundärleitung das Fluid zu dem Verbraucher strömt und überschüssiges Fluid durch die zweite Sekundärleitung in die Hauptversorgungsleitung zurückgeführt wird. Eine Sekundärringleitung ist erheblich besser durchgespült als eine in dieser Hinsicht mangelhafte Stichleitung.

Aus der DE 102 56 584 A1 ist eine Reinstwasserversorgungsanlage für Dialysegeräte bekannt, bei der in der die Hauptversorgungsleitung bildenden Ringleitung eine sekundäre Ringleitung mit ihrem stromaufwärtigen Endabschnitt angeordnet ist, mit einem anschließenden Abschnitt aus der Ringleitung austritt und mit ihrem stromabwärtigen Endabschnitt wieder in die Ringleitung einmündet. Diese Anordnung ist mit verhältnismäßig hohen Herstellungskosten verbunden.

DE 200 13 555 U1 offenbart eine Anlage zur stetigen Erneuerung des Trinkwassers in einer von einer Versorgungsleitung abzweigenden Anschlussleitung. Die Anschlussleitung besteht aus zwei Strängen, die durch eine Innenwand voneinander getrennt sind. Die Innenwand ragt mit einem querschnittlich halbkreisförmigen Endabschnitt in die Versorgungsleitung hinein, wobei die Innenwand quer zur Längsachse der Versorgungsleitung angeordnet ist, so dass an den beidseitig der Innenwand angeordneten Eintrittsöffnungen in die Stränge ein unterschiedlicher Druck herrscht.

Der vorliegenden Erfindung liegt die Aufgabe zugrunde, ein Fluidsystem der betrachteten Art anzugeben, das einen geringen Druckabfall in der Hauptströmung hervorruft und mit niedrigeren Kosten verbunden ist.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass in die Hauptversorgungsleitung wenigstens ein Anschlussblock eingefügt ist, der einen Hauptversorgungsdurchgangskanal aufweist, der einen Abschnitt der Hauptversorgungsleitung bildet, und dass im Winkel zu dem Hauptversorgungsdurchgangskanal, vorzugsweise in einem rechten Winkel, ein Strömungsblock in eine Bohrung des Anschlussblocks eingesetzt ist, der wenigstens einen Sekundärdurchgangskanal aufweist, der mit dem Hauptversorgungsdurchgangskanal in Fluidverbindung steht und an den an der Außenseite des Strömungsblocks eine Sekundärleitung anschließbar ist, und dass der Strömungsblock einen Strömungswiderstandskörper aufweist, der in den Hauptversorgungsdurchgangskanal hinein ragt und bewirkt, dass Fluid in den Sekundärdurchgangskanal eintritt. Der Strömungswiderstandskörper ist dabei einstückig mit dem Strömungsblock ausgebildet.

Es können ein oder zwei Sekundärdurchgangskanäle durch den Strömungsblock hindurchführen, je nachdem, ob eine Sekundärstichleitung oder eine sekundäre Ringleitung zu dem Verbraucher führt.

In einer Ausführungsform verläuft der Sekundärdurchgangskanal/die Sekundärdurchgangskanäle durch den Strömungswiderstandskörper hindurch, wobei ein stromaufwärts bezüglich der Strömung in der Hauptversorgungsleitung ausgebildeter Sekundärdurchgangskanal, der bevorzugt rechtwinklig zu dem Hauptversorgungsdurchgangskanal verläuft, durch eine rechtwinklig abzweigende Bohrung mit dem Hauptversorgungsdurchgangskanal in Strömungsverbindung steht. Dabei befindet sich diese Bohrung bzw. Eintrittsöffnung für das Fluid vorzugsweise etwa in der Mitte des Hauptversorgungsdurchgangskanals.

Wenn bei dieser Ausführungsform ein zweiter Sekundärdurchgangskanal vorgesehen ist, befindet er sich in Strömungsrichtung gesehen im Abstand hinter dem ersten Sekundärdurchgangskanal und verläuft parallel zu diesem. Der zweite Sekundärdurchgangskanal ist mit einer im rechten Winkel abzweigenden Bohrung bzw. Austrittsöffnung versehen, durch die zurückfließendes Fluid wieder in den Hauptversorgungsdurchgangskanal eingeführt wird. Bei dieser Ausführungsform ist der Strömungswiderstandskörper eine Drosselblende, die querschnittlich bevorzugt eine Halbkreisform hat, deren geradlinige Begrenzungsfläche bevorzugt parallel zur Längsachse des Hauptversorgungsdurchgangskanals verläuft. Andere strömungsgünstige Formen der Drosselblende liegen im Rahmen der Erfindung.

In einer anderen Ausführungsform ist als Strömungswiderstandskörper eine Drosselblende vorgesehen, die querschnittlich ein Tragflügelprofil hat, das mit seiner Längserstreckung in Längsrichtung des Hauptversorgungsdurchgangskanals in dem Hauptversorgungskanal angeordnet ist. Bei dieser Ausführungsform verläuft der Sekundärdurchgangskanal/die Sekundärdurch-gangskanäle nicht durch die Drosselblende, sondern endet in der dem Hauptversorgungsdurchgangskanal zugewandten Stirnfläche des Strömungs-blocks, wobei ein Sekundärdurchgangskanal über dem Tragflügelprofil und der andere Sekundärdurchgangskanal mit seiner Öffnung unter dem Tragflügelprofil angeordnet ist. Wegen der Druckdifferenz über und unter dem Tragflügelprofil fließt Fluid durch die sekundäre Ringleitung.

Es liegt im Rahmen der Erfindung, dass in einen Anschlußblock, der in die Hauptversorgungsleitung eingefügt ist, zwei oder mehr Strömungsblöcke eingesetzt sein können, von denen jeweils eine Sekundärstichleitung oder eine Sekundärringleitung abzweigt. Für jeden Verbraucher ist ein zugehöriger Strömungsblock vorgesehen, wobei es sich versteht, dass in die Hauptversorgungsleitung mehrere Anschlussblöcke eingefügt sein können.

Der Strömungsblock ist mit einem vorderen Abschnitt in die Bohrung des Anschlussblocks eingesetzt, während ein rückwärtiger Abschnitt des Strömungsblocks außerhalb des Anschlussblocks verbleibt. Bevorzugt hat zumindest der eingesetzte Abschnitt querschnittlich eine Kreisform und sitzt formschlüssig in der Bohrung des Anschlussblocks. Dabei ist bevorzugt, dass der Strömungsblock in die Bohrung eingesteckt ist. Dies erleichtert die Montage und gegebenenfalls Demontage des Strömungsblocks, wobei die Kreisform der Bohrung die Einstellung des Neigungswinkels des Strömungswiderstandskörpers ermöglichen kann. Die Stirnwand des eingesetzten Abschnitts ist vorzugsweise eben.

Die Hauptversorgungsleitung kann Rohrstangen aus Kunststoff oder beispielsweise VA-Stahl oder aber Schlauchstücke aufweisen, die mit den Anschlussblöcken verbunden sind. Diese Leitungsteile können mit den Anschlussblöcken z.B. verschraubt, verpresst oder mittels Klemmanschlüssen verbunden sein.

Zu letzterem wird mit großem Vorteil vorgeschlagen, dass die Klemmanschlüsse "schwimmend" auf einer Montageplatte befestigt sind. Die Anschlussblöcke können an beiden axialen Enden jeweils mit einem keilartig hinterschnittenen Ansatz versehen sein, während beidseitig anzuschließende Leitungsadapter mit einem ebenso geformten, keilartig hinterschnittenen Ansatz versehen sind. Die beiden mit ebenen Stirnflächen versehenen querschnittlich keilartigen Ansätze, die sich kreisförmig um den gesamten Umfang erstrecken, werden mittels zweier Klemmhalbschalen aneinander gepresst, die an ihrem inneren Rand eine umlaufende V-Nut haben, die formschlüssig die zusammengefügten keilartig hinterschnittenen Ansätze des Anschlussblocks und des Leitungsadapters aufnimmt und zusammen presst. Die beiden Klemmhalbschalen werden durch Schrauben miteinander verbunden.

Die untere Klemmhalbschale hat bodenseitig zwei vorstehende Bolzen oder Schrauben, die in zwei zusammengehörende Löcher einer Montageplatte einsetzbar sind, wobei die Löcher zusammen gesetzt sind aus einem etwa kreisrunden Abschnitt größeren Durchmessers und einem anschließenden länglichen Abschnitt geringerer Breite. Die untere Klemmhalbschale wird in der Weise an der Montageplatte befestigt, dass die beiden bodenseitigen Schraubenköpfe durch die kreisrunden Abschnitte der Löcher hindurchgesteckt werden, wobei die Klemmhalbschale so gedreht wird, dass die Schäfte der Schrauben in die Abschnitte geringerer Breite wandern. Diese bajonettähnliche Befestigung ermöglicht es, dass die Klemmhalbschale in der Befestigungslage in einem gewissen Ausmaß beweglich auf der Montageplatte verrastet ist. Diese schwimmende Lagerung ist in der DE 2006 028 764 B3 des Anmelders der vorliegenden Anmeldung offenbart.

Diese schwimmende Lagerung bewirkt, dass Verspannungen und Wärmeausdehnungen in der Hauptversorgungsleitung ausgeglichen werden können, so dass es hierdurch nicht zu Leckagen kommen kann. Außerdem können hieraus einwirkende Kräfte auf die Sekundärleitungen vermieden werden. Dies ist z.B. bei der Heißreinigung des Leitungssystems sehr vorteilhaft, da die Hauptversorgungsleitung hierdurch eine Ausdehnungsmöglichkeit erhält.

Die Hauptversorgungsleitung kann aus unterschiedlichen Materialien bestehen, die entweder direkt an den Anschlussblock oder mittels Adapter anschließbar sind. Hierbei sind auch Schlauchtüllenanschlüsse und Klebeanschlüsse grundsätzlich möglich.

Die Verwendung von Klemmanschlüssen an die Anschlussblöcke hat den Vorteil, dass diese bei Bedarf einfach austauschbar sind.

Nach einem weiteren Vorschlag der Erfindung kann der wenigstens eine Strömungsblock eine Einrichtung zur Verhinderung des Aufwuchses von Mikroorganismen oder des Aufbaus von Biofilm aufweisen. Hierzu kann durch eine Seitenwand des bei dieser Ausführungsform zweckmäßigerweise etwa würfelförmigen Strömungsblocks eine Bohrung zu einer Sekundärdurchgangsbohrung führen, in die eine UV-Quelle einsetzbar ist. Diese UV-Quelle ist an einem Stopfen befestigt, mit dem die Bohrung verschlossen wird. Die UV-Quelle kann als UV-LED ausgebildet sein. Es ist auch möglich, ein aktives antimikrobielles Material in die Antikontaminationskammer einzusetzen. Die Kammer kann bei Einsatz einer UV-LED auch reflektierend ausgeführt sein. Ebenso ist es möglich, dass nach dem Einsatz einer Ringleitungs-Ozondesinfektion die UV-Quelle die Neutralisierung des giftigen Ozons herbeiführt.

Nach einem weiteren Vorschlag der Erfindung kann der Strömungsblock mit einer Einrichtung zur Erfassung der Strömung versehen sein, die die Strömung in dem Sekundärdurchgangskanal erfasst, die das Fluid zu dem Verbraucher führt. Hierdurch kann eine ungenügende Durchströmung der Sekundärleitung festgestellt werden, was insbesondere nach einer Desinfektion des Fluidsystems sehr wichtig ist, da z.B. bei einer Verstopfung der sekundären Schlauchleitung oder im Falle eines scharfen Knicks der Leitung das Entfernen von Rückständen des Desinfektionsmittels beeinträchtigt sein kann. Dies könnte zu schwerwiegenden Schäden beim Verbraucher führen. Der elektronische Strömungswächter zeigt die Strömungsgeschwindigkeit bzw. einen definierten Fluss-Grenzwert in der Sekundärleitung an.

Außerdem können in dem Strömungsblock weitere mess- und regelungstechnische Einrichtungen wie Sensoren zur Messung von Zustandsgrößen, Durchsätzen und Stoffeigenschaften untergebracht sein.

Ebenso ist eine Beschichtung des Strömungswiderstandskörpers mit einer Antikontaminationsschicht wie z.B. aus Silber möglich.

Ein bevorzugtes Anwendungsgebiet der Erfindung ist die Dialyse, wobei das Fluidsystem Reinstwasser (Permeat) von einer Umkehrosmose durch eine Ringleitung und von dort durch abzweigende Sekundärleitungen zu Dialysegeräten führt. Dabei werden mehrere Anschlussblöcke in die Ringleitung eingefügt, um die Dialysegeräte mit Permeat zu versorgen. Die Drosselblenden der Strömungswiderstandskörper rufen nur einen sehr geringen Druckabfall in der Ringleitung hervor, so dass auch die in Strömungsrichtung hinteren Dialysegeräte ausreichend mit Permeat versorgt werden. In Folge der besonderen Klemmanschlüsse, die bevorzugt verwendet werden, ist die Ringleitung totraumfrei, so dass mikrobiologisches Wachstum weitestgehend vermieden ist.

Nach einem weiteren Vorschlag der Erfindung ist die Ringleitung mit den Anschlussblöcken durch eine Wand von den zugehörigen Verbrauchern getrennt, und die Sekundärleitungen verlaufen durch diese Wand hindurch zu den Dialysegeräten. Erfindungsgemäß sind zur Aufnahme und Halterung der die Sekundärleitungen bildenden Schläuche Schlauchhalter an der Wand befestigt, die eine Bodenberührung der Schläuche verhindern. Die Schlauchhalter haben zweckmäßigerweise Öffnungen, in die die Schläuche eingeklemmt werden können, wobei auch der geräteseitige Endabschnitt der Schläuche nach Diskonnektion von dem Dialysegerät in den Schlauchhalter eingehängt werden sollte.

Die Schlauchhalter verhindern, dass die Schläuche auf dem Boden liegen und bei evtl. Reinigungsmaßnahmen des Bodens verschmutzt werden.

Es kann auch eine Roll- bzw. Zugvorrichtung vorgesehen sein, die die nicht benötigte Schlauchlänge aufwickelt.

Auch können in diese Halterung elektrische Versorgungsleitungen mit eingehängt werden.

Weitere Einzelheiten der Erfindung ergeben sich aus der nachfolgenden Beschreibung einiger bevorzugter Ausführungsformen sowie anhand der Zeichnungen. Dabei zeigen:
- Figur 1: einen Ringleitungs-Anschlussblock mit PEX-Anschlüssen in verschiedenen Darstellungen;
- Figur 2: einen Ringleitungs-Doppelanschlussblock mit Klemmanschlüssen in verschiedenen Darstellungen;
- Figur 3: einen prismatischen Ringleitungs-Anschlussblock mit Klemmanschlüssen und einer Dekontaminationskammer und Sensorkammer;
- Figur 4: einen schematischen Zusammenbau des Fluidsystems mit einer Trennwand z.B. einer Dialysestation.

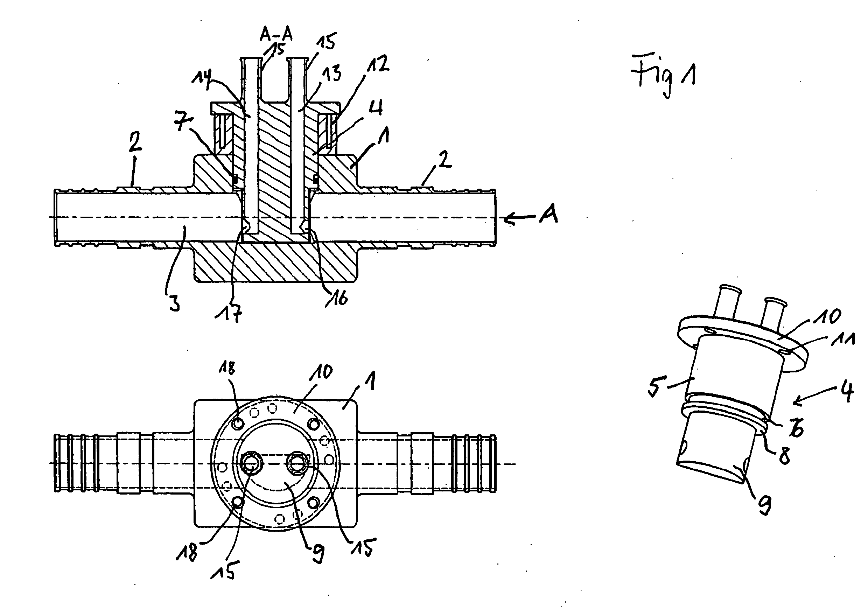

In Figur 1 ist eine erste Ausführungsform eines Anschlussblocks 1 dargestellt, der mit beidseitigen PEX-Anschlüssen 2 versehen ist. Entgegen der Darstellung in Figur 1 können die PEX-Anschlüsse in seitliche Bohrungen des Anschlussblocks 1 eingesteckt und mit Ringdichtungen abgedichtet werden. Der Anschlussblock 1 enthält einen Ringleitungskanal 3, der sich in den PEX-Anschlüssen 2 fortsetzt. Auf die PEX-Anschlüsse 2 können beispielsweise Schlauchstücke aufgesetzt sein.

Ein Strömungsblock 4 ist senkrecht zur Längsachse des Durchgangskanals 3 in den Anschlussblock 1 eingesetzt. Der Strömungsblock 4 enthält einen zylindrischen Abschnitt 5, der in eine entsprechende Bohrung des Anschlussblocks 1 eingesteckt ist, wobei eine in eine Nut 6 eingesetzte Ringdichtung 7 dafür sorgt, dass kein Fluid aus der Bohrung austreten kann. Der zylindrische Abschnitt 5 endet in der Darstellung der Figur 1 unten in einer ebenen Stirnwand 8, an der eine Drosselblende 9 angesetzt ist, die sich quer durch den Durchgangskanal 3 im wesentlichen bis zur gegenüberliegenden Kanalwand erstreckt. Die Drosselblende 9 hat etwa die Querschnittsform eines Halbkreises, wie die Darstellung links unten in Figur 1 in gestrichelter Linie zeigt.

An dem gegenüberliegenden Ende des zylindrischen Abschnitts 5 befindet sich ein ringförmiger Flansch 10 mit Durchgangsbohrungen 11 für Befestigungsschrauben, die in einen zwischen der Oberseite des Anschlussblocks 1 und der Unterseite des Flansches 10 angeordneten Ring 12 eingeschraubt werden, um den Strömungsblock 4 sicher an dem Anschlussblock 1 zu befestigen. Bei geeigneter Konstruktion und Materialwahl kann der Strömungsblock 4 auch ohne Ring 12 am Anschlussblock befestigt werden.

Durch die Drosselblende 9 und den Strömungsblock 4 verlaufen zwei Sekundärdurchgangskanäle 13 und 14, die mit Anschlussstutzen 15 von dem Ringflansch 10 vorstehen, mit denen nicht dargestellte Sekundärleitungen, allgemein in Form von Schläuchen, befestigt werden. An den anderen Enden gehen die Sekundärdurchgangskanäle 13 und 14 in abgewinkelte Eintritts- und Austrittsöffnungen 16 und 17 über. Wenn das Fluid - im Falle einer Ringleitung einer Dialysestation das Permeat - in Richtung eines Pfeils A durch den Durchgangskanal strömt, tritt Permeat infolge des Strömungswiderstands durch die Öffnung 16 in den Sekundärdurchgangskanal 13 ein, wobei überschüssiges Permeat durch den Sekundärdurchgangskanal 14 und die Austrittsöffnung 17 wieder in den Durchgangskanal 3 zurück geführt wird.

Die Befestigungsschrauben des Strömungsblocks 4 sind in der Darstellung links unten in Figur 1 mit dem Bezugszeichen 18 bezeichnet.

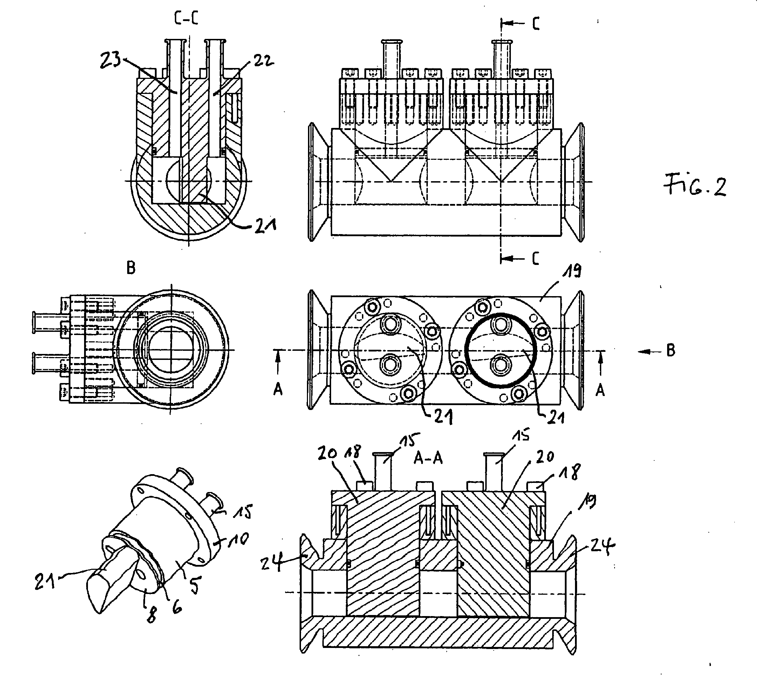

Bei der Ausführungsform der Figur 2 sind in einen Anschlussblock 19 zwei Strömungsblöcke 20 nebeneinander eingesetzt. Die Strömungsblöcke 20 tragen an ihrer ebenen Stirnfläche 8 eine Drosselblende 21, die querschnittlich ein Tragflügelprofil hat, wie in der mittleren rechten Darstellung der Figur 2 gestrichelt gezeigt ist. Bei dieser Ausführungsform enden die SekundärDurchgangskanäle 22 und 23 in der Stirnwand 8 des Strömungsblocks 20, und zwar zu beiden Seiten der tragflügelartigen Drosselblende 21, so dass unterschiedliche Druckverhältnisse in dem strömenden Fluid an den Ein- bzw. Austrittsöffnungen der Sekundärdurchgangskanäle 22 und 23 herrschen, durch die das Fluid in einen Kanal eintritt und überschüssiges Fluid aus dem anderen Kanal wieder austritt.

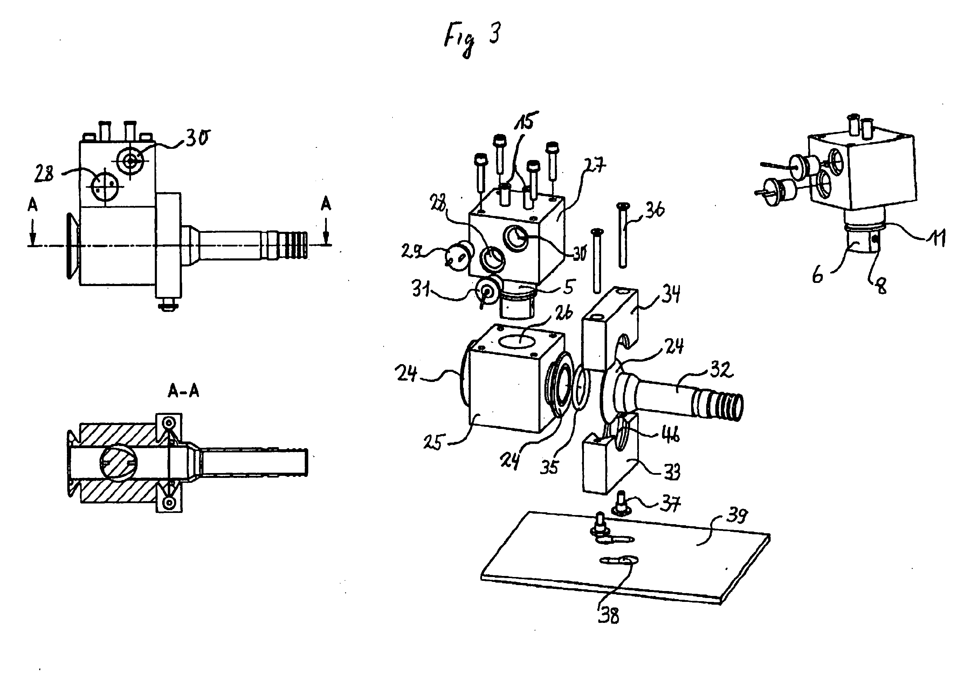

Der Anschlussblock 19 ist mit angeformten Klemm-Halbschalen 24 an beiden Seiten versehen, wobei dieser Klemmanschluss in seinen Einzelheiten in Figur 3 dargestellt ist.

Figur 3 zeigt einen prismaförmigen Anschlussblock 25 in dessen Bohrung 26 der zylindrische Abschnitt 5 eines Strömungsblocks einsteckbar ist, der mit einem ebenfalls prismaförmigen Körper 27 auf der Oberseite des Strömungsblocks 25 aufliegt. Der Strömungsblock ist mit einer Drosselblende 9 versehen, die wie bei der Ausführungsform der Figur 1 in den Durchgangskanal hineinragt. Durch den prismaförmigen Körper 27 verlaufen die beiden Sekundärdurchgangskanäle, die mit Anschlussstutzen 15 an der Oberseite des Körpers 27 austreten.

In dem prismaförmigen Körper 27 führen zwei Bohrungen zu den sekundären Durchgangskanälen, wobei in eine Bohrung 28 eine mit einem Stopfen versehende UV-Quelle 29 eingesetzt ist, während in die andere Bohrung 30 ein mit einem Stopfen versehender Strömungswächter 31 eingesetzt ist. Die UV-Quelle ist jeweils in die zum Verbraucher führende Strömung einzusetzen, deshalb kann nach Wahl der Strömungskörper zwischen Bohrung 27 und 28 gewechselt werden.

An dem Anschlussblock 25 ist beidseitig jeweils ein keilartig hinterschnittener Ansatz 24 angesetzt, der jeweils mit einem Leitungsadapter 32 verbindbar ist, der einen identischen Ansatz 24 enthält. Die querschnittlich keilartigen Ansätze 24 werden durch zwei Klemmhalbschalen 33 und 34 fest miteinander verbunden, die an ihrem inneren Rand eine umlaufende V-Nut 46 haben, die formschlüssig die zusammengefügten keilartig hinterschnittenen Ansätze des Anschlussblocks und des Leitungsadapters aufnimmt und zusammen presst. Eine Ringdichtung 35 in den Stirnflächen der keilartig hinterschnittenen Ansätze 24 sorgt für einen flüssigkeitsdichten Anschluss. Die Klemmhalbschalen 33 und 34 werden durch Schrauben 36 miteinander verschraubt.

Von der Unterseite der unteren Klemmhalbschale 33 stehen zwei Kopfabschnitte von Schrauben 37 vor, die in leicht bogenförmig gekrümmte Langlöcher 38 einsetzbar sind, die jeweils eine verbreiterte Eintrittsöffnung und einen anschließenden schmaleren Abschnitt aufweisen, aus denen die Schrauben 37 nicht austreten können. Die Ausbildung ist so getroffen, dass diese Halterung der Klemmhalbschalen 33 und 34 an der Montageplatte 39 "schwimmend" ist, d.h. die Klemmhalbschalen sind in einem gewissen Ausmaß auf der Montageplatte 39 bewegbar, so dass sie z.B. Längenausdehnungen infolge Erwärmung des Leitungssystems bei der Heißreinigung folgen können.

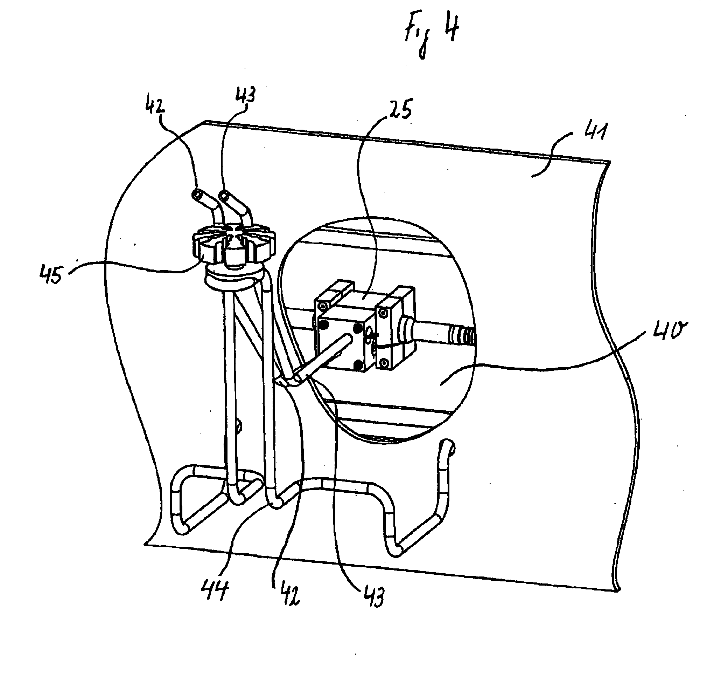

Figur 4 zeigt die Anordnung eines Anschlussblocks 25 auf einer Konsole 40 einer Trennwand 41 einer Dialysestation. Eine Trennwand 41 schirmt das dahinter befindliche Leitungssystem ab, wobei die Sekundärleitungen 42, 43 durch Öffnungen in der Wand 41 hindurchführen. An der Wand 41 ist ein Gestell 44 mit einer Kabel- und Schlauchfixiereinrichtung 45 befestigt, in der die sekundären Ringleitungen 42 und 43 gehalten sind. Bei einer Diskonnektierung dieser Schläuche können diese in die Kabel- und Schlauchfixiereinrichtung eingehängt werden, damit sie den Boden nicht berühren.

Es wird betont, dass die Erfindung nicht auf die beschriebenen und dargestellten Ausführungsformen beschränkt ist. Vielmehr sind alle in den Zeichnungen und in der Beschreibung offenbarten Merkmale auf jede sinnvolle Weise einzeln miteinander kombinierbar.

## Patentansprüche

1. Fluidsystem mit einer Hauptversorgungsleitung und wenigstens einer davon abzweigenden, zu Verbrauchern führenden Sekundärleitung, wobei in die Hauptversorgungsleitung wenigstens ein Anschlussblock (1,19,25) eingefügt ist, der einen Hauptversorgungsdurchgangskanal (3) aufweist, der einen Abschnitt der Hauptversorgungsleitung bildet, und wobei seitlich, vorzugsweise im rechten Winkel zu dem Hauptversorgungs-durchgangskanal (3), ein Strömungsblock (4, 20) in eine Bohrung des Anschlussblocks eingesetzt ist, der wenigstens einen Sekundärdurch-gangskanal (13,14), an den eine Sekundärleitung (42,43) anschließbar ist, und einen Strömungswiderstandskörper (9,21) aufweist, der in den Hauptversorgungsdurchgangskanal hineinragt
**dadurch gekennzeichnet,**
**dass** der Strömungswiderstandskörper (9, 21) eine Drosselblende ist, die eine strömungsgünstige Form hat, indem sie querschnittlich ein Tragflügelprofil oder etwa eine Halbkreisform hat, deren ebene Fläche parallel zur Längsachse des Hauptversorgungsdurchgangskanals (3) liegt.

2. Fluidsystem nach Anspruch 1,
**dadurch gekennzeichnet,**
**dass** der Strömungsblock (4,20) in den Anschlussblock (1, 19, 25) eingesteckt ist.

3. Fluidsystem nach einem der Ansprüche 1 oder 2,
**dadurch gekennzeichnet,**
**dass** die Hauptversorgungsleitung Rohre aus Kunststoff oder VA-Stahl oder Schlauchstücke aufweist.

4. Fluidsystem nach Anspruch 3,
**dadurch gekennzeichnet,**
**dass** die Rohrstangen oder Schlauchstücke mit dem wenigstens einen Anschlussblock mittel Klemmanschlüssen (24,33,34) lösbar verbunden sind.

5. Fluidsystem nach Anspruch 4,
**dadurch gekennzeichnet,**
**dass** die Klemmanschlüsse (24,33,34) schwimmend auf einer Montageplatte (39) befestigt sind.

6. Fluidsystem nach einem der Ansprüche 1 bis 5,
**dadurch gekennzeichnet,**
**dass** die Sekundärdurchgangskanäle (13,14) mit von dem Strömungsblock (4,30) vorstehenden Anschluss-Stutzen (15) verbunden sind, an denen Schläuche der Sekundärleitungen (42,43) durch Steck-, Schraub-, Klemm- oder Schlauchan-schlüsse anbringbar sind.

7. Fluidsystem nach einem der Ansprüche 1 bis 6,
**dadurch gekennzeichnet,**
**dass** eine Wand (41) die Hauptversorgungsleitung mit dem wenigstens einen Anschlussblock (1, 19, 25) von den zugehörigen Verbrauchern trennt, dass die Sekundärleitungen (42,43) durch die Wand (41) hindurch verlaufen und
**dass** zur Aufnahme und Halterung der Schläuche der Sekundärleitungen (42,43) eine Schlauchfixiereinrichtung (45) an der Wand (41) befestigt ist, die eine Bodenberührung der Schläuche verhindert.

8. Fluidsystem nach einem der Ansprüche 1 bis 7,
**dadurch gekennzeichnet,**
**dass** der Strömungsblock eine Einrichtung (29) zur Verhinderung des Aufwuchses von Mikroorganismen oder des Aufbaus von Biofilm aufweist.

9. Fluidsystem nach Anspruch 8,
**dadurch gekennzeichnet,**
**dass** die Einrichtung (22) eine UV-Lichtquelle und/oder aktives antimikrobielles Material aufweist.

10. Fluidsystem nach einem der Ansprüche 1 bis 9,
**dadurch gekennzeichnet,**
**dass** der Strömungsblock eine Einrichtung (30) zur Erfassung der Strömung in der wenigstens einen Sekundärleitung aufweist.

11. Fluidsystem nach einem der Ansprüche 1 bis 10,
**dadurch gekennzeichnet,**
**dass** die Hauptversorgungsleitung eine Ringleitung einer Dialysestation ist, die Reinstwasser (Permeat) von einer Umkehrosmose durch Sekundärleitungen zu Dialysegeräten führt.

## Claims

1. A fluid system with a main supply line and at least one secondary line, branching off from it, leading to consumers, wherein at least one connecting block (1, 19, 25) is inserted into the main supply line, which includes a main supply flow passage (3), which constitutes a section of the main supply line, and wherein inserted laterally, preferably at right-angles to the main supply flow passage (3), into a bore in the connecting block there is a flow block (4, 20), which includes at least one secondary flow passage (13, 14), to which a secondary line (42, 43) is connectable, and a flow resistance body (9, 29), which extends into the main supply flow passage, **characterised in that** the flow resistance body (9, 29) is an orifice plate, which has a hydrodynamic shape **in that** it has an aerofoil profile in cross-section or approximately a semi-circular shape, whose flat surface lies parallel to the longitudinal axis of the main supply flow passage (3).

2. A fluid system as claimed in Claim 1, **characterised in that** the flow block (4, 20) is inserted into the connecting block (1, 19, 25).

3. A fluid system as claimed in one of Claims 1 or 2, **characterised in that** the main supply line has pipes of plastic or high-alloyed steel or tube pieces.

4. A fluid system as claimed in Claim 3, **characterised in that** the tubular bars or tube pieces are releasably connected to the at least one connecting block by means of clamp connectors (24, 33, 34).

5. A fluid system as claimed in Claim 4, **characterised in that** the clamp connectors (24, 33, 34) are floatingly fastened to a mounting plate (39).

6. A fluid system as claimed in one of Claims 1 to 5, **characterised in that** the secondary flow passages (13, 14) are connected to connecting pieces (15), which project from the flow block (4, 30) and to which hoses of the secondary lines (42, 43) may be fitted by plug, screw, clamp or hose connectors.

7. A fluid system as claimed in one of Claims 1 to 6, **characterised in that** a wall (41) separates the main supply line with the at least one connecting block (1, 19, 25) from the associated consumers, that the secondary lines (42, 43) extend through the wall (41) and that for the purpose of receiving and holding the hoses of the secondary lines (42, 43) a hose fixing device (45) is secured to the wall (41), which prevents contact with the ground by the hoses.

8. A fluid system as claimed in one of Claims 1 to 7, **characterised in that** the flow block includes a device (29) for preventing the growth of microorganisms or the growth of a biofilm.

9. A fluid system as claimed in Claim 8, **characterised in that** the device (22) includes a UV light source and/or active antimicrobial material.

10. A fluid system as claimed in one of Claims 1 to 9, **characterised in that** the flow block includes a device (30) for detecting flow in the at least one secondary line.

11. A fluid system as claimed in one of Claims 1 to 10, **characterised in that** the main supply line is an annular conduit of a dialysis station, which conducts highly pure water (permeate) from a reverse osmosis process through secondary lines to dialysis devices.

## Revendications

1. Système fluidique comprenant une conduite d'alimentation principale et au moins une conduite secondaire bifurquant de cette dernière, menant aux consommateurs, sachant qu'au moins un bloc de raccordement (1, 19, 25) est inséré dans la conduite d'alimentation principale, lequel bloc de raccordement présente un canal de passage d'alimentation principale (3), qui forme un tronçon de la conduite d'alimentation principale, et sachant qu'est encastré latéralement, de préférence dans l'angle gauche par rapport au canal de passage d'alimentation principale (3), dans un alésage du bloc de raccordement, un bloc d'écoulement (4, 20) présentant au moins un canal de passage secondaire (13, 14), auquel peut être raccordée une conduite secondaire (42, 43), et un corps de résistance d'écoulement (9, 21), lequel fait saillie à l'intérieur du canal de passage d'alimentation principale,
**caractérisé en ce**
**que** le corps de résistance d'écoulement (9, 21) est un diaphragme d'étranglement, qui présente une forme favorable à l'écoulement, en ce qu'elle comporte dans la section transversale un profilé porte-aube ou présente à peu près une forme semi-circulaire, dont la surface plane est parallèle par rapport à l'axe longitudinal du canal de passage d'alimentation principale (3).

2. Système fluidique selon la revendication 1,
**caractérisé en ce**
**que** le bloc d'écoulement (4, 20) est encastré dans le bloc de raccordement (1, 19, 25).

3. Système fluidique selon l'une quelconque des revendications 1 ou 2,
**caractérisé en ce**
**que** la conduite d'alimentation principale présente des tuyaux en plastique ou en acier inoxydable ou des parties de tuyau flexible.

4. Système fluidique selon la revendication 3,
**caractérisé en ce**
**que** les tiges de tuyau ou les parties de tuyau flexible sont reliées de manière amovible au bloc de raccordement au moins au nombre de un au moyen de dispositif de raccordement par serrage (24, 33, 34).

5. Système fluidique selon la revendication 4,
**caractérisé en ce**
**que** les dispositifs de raccordement par serrage (24, 33, 34) sont fixés de manière flottante sur une plaque de montage (39).

6. Système fluidique selon l'une quelconque des revendications 1 à 5,
**caractérisé en ce**
**que** les canaux de passage secondaires (13, 14) sont reliés à des tubulures de raccordement (15) faisant saillie du bloc d'écoulement (4, 30), au niveau desquelles peuvent être installés des tuyaux flexibles des conduites secondaires (42, 43) grâce à des dispositifs de raccordement par enfichage, par vissage, par serrage ou pour tuyau flexible.

7. Système fluidique selon l'une quelconque des revendications 1 à 6,
**caractérisé en ce**
**qu'**une paroi (41) sépare la conduite d'alimentation principale dotée du bloc de raccordement (1, 19, 25) au moins au nombre de un des consommateurs associés,
en ce que les conduites secondaires (42, 43) s'étendent à travers la paroi (41), et
en ce qu'aux fins du logement et du support des tuyaux flexibles des conduites secondaires (42, 43) est fixé au niveau de la paroi (41) un système de fixation de tuyau flexible (45), lequel empêche que les tuyaux flexibles ne touchent le sol.

8. Système fluidique selon l'une quelconque des revendications 1 à 7,
**caractérisé en ce**
**que** le bloc d'écoulement présente un système (29) servant à empêcher la prolifération de micro-organismes ou l'apparition de biofilm.

9. Système fluidique selon la revendication 8,
**caractérisé en ce**
**que** le système (22) présente une source de lumière UV et/ou un matériau actif anti-microbien.

10. Système fluidique selon l'une quelconque des revendications 1 à 9,
**caractérisé en ce**
**que** le bloc d'écoulement présente un système (30) servant à détecter l'écoulement dans la conduite secondaire au moins au nombre de une.

11. Système fluidique selon l'une quelconque des revendications 1 à 10,
**caractérisé en ce**
**que** la conduite d'alimentation principale est une conduite annulaire d'une station de dialyse, laquelle achemine de l'eau déminéralisée (perméat) en provenance d'une osmose inversée à travers les conduites secondaires en direction d'appareils de dialyse.
